(19) Europäisches Patentamt
European Patent Office
Office européen des brevets

(11) Veröffentlichungsnummer: **0 289 604**
**A1**

(12) # EUROPÄISCHE PATENTANMELDUNG

veröffentlicht nach Art. 158 Abs. 3 EPÜ

(21) Anmeldenummer: **87900733.4**

(51) Int. Cl.[4]: **C 07 D 211/90**

(22) Anmeldetag: **10.11.86**

Daten der zugrundeliegenden internationalen Anmeldung:
(86) Internationale Anmeldenummer:
**PCT/SU 86/00113**
(87) Internationale Veröffentlichungsnummer:
**WO 88/03529 (19.05.88 88/11)**

(43) Veröffentlichungstag der Anmeldung: **09.11.88**
**Patentblatt 88/45**

(84) Benannte Vertragsstaaten: **CH DE FR GB IT LI**

(71) Anmelder: **INSTITUT ORGANICHESKOGO SINTEZA AKADEMII NAUK LATVIISKOI SSR, ul. Aizkraukles, 21, Riga 226006 (SU)**

(72) Erfinder: **KRAUZE, Aivars Arnisovich, ul. Slokas, 205-41, Riga, 226069 (SU)**
Erfinder: **PELCHER, Juris Edgarovich, ul. Kirova, 89-19, Riga, 226011 (SU)**
Erfinder: **VITOLIN, Rasma Oskarovna, ul. Suvorova, 117-13, Riga, 226001 (SU)**
Erfinder: **SELGA, Marite Janovna, ul. Kursas, 1 Rizhsky raion, selo Garkalne, 229065 (SU)**
Erfinder: **PETERSONE, Inta Ottovna, ul. Melnsila, 28-5, Riga, 226046 (SU)**
Erfinder: **KALME, Zenta Alfredovna, ul. Andromedas Gatve, 2-32, Riga, 226080 (SU)**
Erfinder: **KIMENIS, Agris Adolfovich, ul. Staitseles, 15-208, Riga, 226084 (SU)**
Erfinder: **DUBUR, Gunar Janovich, ul. Ierikju, 43-2, Riga, 226059 (SU)**

(74) Vertreter: **von Füner, Alexander, Dr. et al, Patentanwälte v. Füner, Ebbinghaus, Finck Mariahilfplatz 2 & 3, D-8000 München 90 (DE)**

(54) **2-METHYLMERCAPTO-3-CYANO-4-(O-DIFLUORMETHOXYPHENYL)-6-PHENYL-1,4-DIHYDROPYRIDINE.**

(57) 2-Methylmerkapto-3-zyano-4-(o-difluormethoxyphenyl)-6-phenyl-l,4-dihydropyridin wird durch die Formel

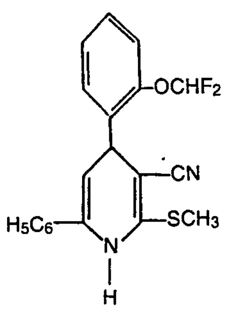

gekennzeichnet.

Diese Verbindung besitzt eine hypotensive Wirksamkeit.

EP 0 289 604 A1

# 2-METHYLMERKAPTO-3-ZYANO-4-(O-DIFLUORMETHOXYPHENYL)-6--PHENYL-I,4-DIHYDROPYRIDIN

## Gebiet der Technik

Die vorliegende Erfindung bezieht sich auf ein Gebiet der organischen Synthese und betrifft insbesondere I,4-Dihydropyridinderivate und insbesondere 2-Methylmerkapto-3-zyano-4-(o-difluormethohyphenyl)-6-phenyl-I,4-dihydropyridin.

Die I,4-Dihydropyridinderivate besitzen eine hypotensive Wirkung und einige davon werden in der Medizin verwendet.

## Vorheriger Stand der Technik

Das bekannteste I,4-Dihydropyridinderivat, das eine hypotensive und koronarerweiternde Wirkung besitzt, ist 2,6-Dimethyl-3,5-dimethoxykarbonyl-4-(o-nithophenyl)-I,4-dihydropyridin (Nifedipine) (Arzneimittel-Forschung, v. 32, I982, E.Kusano, J.Asano, K.Takeda. J.Matsumoto, A.Ebihara. "Hypotensive effect of Nifedipine in Hypertensive Patients with Chronic Renal Failure", pp. I575-I580).

Zwar findet Nifedipine eine breite Verwendung in der medizinischen Praxis, ist es jedoch lichtunbeständig und dabei hochtoxisch.

Ein anderes bekanntes I,4-Dihydropyridinderivat ist 2,6-Dimethyl-3,5-dimethoxykarbonyl-4-(o-difluormethoxyphenyl)-I,4-dihydropyridin (Riodipine), das auch eine hypotensive Wirksamkeit aufweist (SU-PS, 7064IO, IPK C 07 D 2II/90, A 6I K 3I/435, veröffentlicht in I979). Es muss hervorgehoben werden, dass Riodipine eine hohe hypotensive und koronarerweiternde Wirkung besitzt, dabei ist jedoch dessen hypotensive Wirkung nicht anhaltend genug.

## Offenbarung der Erfindung

Die Aufgabe der vorliegenden Erfindung bestand in der Forschung nach solch einem I,4-Dihydropyridinderivat, das eine anhaltendere hypotensive Wirkung besitzt und wenig toxisch ist.

Die gestellte Aufgabe wurde dadurch gelöst, dass ein I,4-Dihydropyridinderivat entwickelt wurde, das erfindungsgemäss 2-Methylmerkapto-3-zyano-4-(o-difluormethoxyphenyl)-6-phenyl-I,4-dihydropyridin der Formel

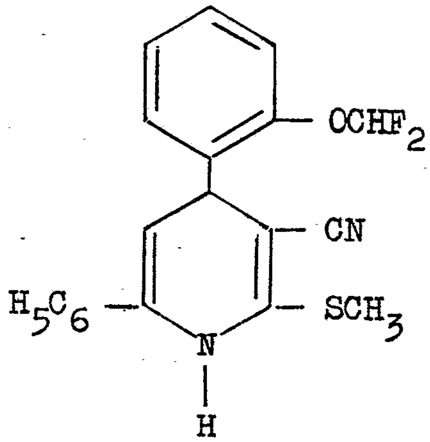

darstellt. Diese Verbindung ist neu. Es wurde von uns festgestellt, dass die genannte Verbindung eine hohe hypotensive Wirkung besitzt, die während einer längeren Zeit im Vergleich zu den oben genannten Verbindungen anhält.

Ausserdem wurde festgestellt, dass die genannte Verbindung eine niedrigere Toxizität im Vergleich zur bekannten Verbindung besitzt: $LD_{50} > 5000$ mg/kg; $LD_{50}$ für Nifedipine beträgt I85 mg/kg und für Riodipine 395 mg/kg.

Die genannte Verbindung stellt eine kristalline Substanz vom I28 bis I30°C Schm.p. dar. Sie ist in Methyl-, Äthylalkohol, Azeton, Chloroform gut löslich und in Wasser praktisch unlöslich. Diese Verbindung ist sowohl in kristallinem Zustand als auch in Form von Lösungen in organischen Lösungsmitteln stabil.

Die genannte Verbindung wird durch Umsetzung von o-Difluormethoxybenzylidenazetophenon mit Zyanothioazetamid in Gegewart von Pyridin hergestellt. Die Reaktion wird im Medium eines organischen Lösungsmittels bei Zimmertemperatur und unter Normaldruck durchgeführt. Das erhaltene Piperidinsalz von I,4-Dihydropyridin wird mit Dimethylsulfat unmittelbar im Reaktionsgemisch oder nach dessen Isolierung behandelt. Man erhält das Produkt in einer hohen Ausbeute.

Somit ist das Verfahren zur Herstellung der genannten Verbindung einfach, weil die Reaktion unter milden Bedingungen vor sich geht. Die Ausgangskomponenten sind bekannt und zugänglich /Zeitschrift "Chemie der heterozyklischen

Verbindungen" (Khimiya geterotsiklicheskikh soedineny) Nr. 3, I98I, A.A.Krause und andere "Synthese und einige Reaktionen von 3-Zyanopyridin-2-thionen", S. 377/.

Die pharmakologische Untersuchung der Wirkung von 2-Methylmerkapto-3-zyano-4-(o-difluormethoxyphenyl)-6-phenyl--I,4-dihydropyridin auf das kardiovaskuläre System wurde im Vergleich zum in der Klinik breit verwendbaren Arzneimittel Nifedipine sowie zum nahen Strukturanalogon Riodipine durchgeführt.

In den Versuchen an spontan hypertensiven Ratten (SHR) ruft die einmalige Einführung der Verbindung in den Magen in einer Dosis von IO mg/kg den Effekt der Senkung des systolischen arteriellen Blutdrucks 30 Minuten nach der Einführung hervor, dabei wird die hypotensive Wirkung innerhalb von 48 Stunden nachgewiesen. In einer ähnlichen Dosis übersteigt Nifedipine I Stunde nach der Einführung die genannte Verbindung dem Wert der hypotensiven Wirkung nach; nach 6 Stunden hört die Wirkung von Nifedipine ebenso wie die von Riodipine auf und die erfindungsgemässe Verbindung weist noch einen ausgeprägten Effekt auf - der systolische Blutdruck ist um 2I% herabgesetzt. Die Angaben der pharmakologischen Untersuchung sind in der Tabelle I angeführt.

Tabelle I

Hypotensiver Effekt von erfindungsgemässer Verbindung, Nifedipine und Riodipine in einer Dosis von IO mg/kg

| Verbindung | Der hypotensive Effekt, % an SHR | | | |
|---|---|---|---|---|
| | nach I Stunden | nach 6 Stunden | nach 24 Stunden | nach 48 Stunden |
| Die erfindungsgemässe Verbindung | I7 | 24 | 2I | 6 |
| Nifedipine | 34 | 23 | 0 | 0 |
| Riodipine | 22 | I5 | 0 | 0 |

Somit übersteigt die erfindungsgemässe Verbindung bedeutend Nifedipine und Riodipine der hypotensiven Wirkungsdauer nach.

Bei der wiederholten Einführung den spontan hypertensiven Ratten der erfindungsgemässen Verbindung je zu IO mg/kg in den Magen einmal pro Tag innerhalb von IO Tagen wird ein stabiler hypotensiver Effekt nachgewiesen. Zum IO. Einführungstag war der systolische Blutdruck um 26% herabgesetzt. Nach dem Aufhören der Einführung der Verbindung erreichte der arterielle Blutdruck das Ausgangsniveau innerhalb von 3 Tagen.

Nifedipine übt bei der IO Tage langen Einführung eine ähnliche Wirkung auf den systolischen Blutdruck in einer Dosis von 5 mg/kg aus, dabei ist es jedoch toxischer.

Die Untersuchung der akuten Toxizität der erfindungsgemässen Verbindung an weissen gattungslosen Mäusen bei der intraperitonealen Einführung hat ergeben, dass diese im Vergleich zu den genannten Verbindungen weniger toxisch ist. Die Untersuchungsangaben sind in der Tabelle 2 angeführt.

Tabelle 2

Akute Toxizität

| Verbindung | $LD_{50}$, mg/kg |
|---|---|
| Die erfindungsgemässe Verbindung | > 5000 |
| Nifedipine | I85 (II9-287) |
| Riodipine | 395 (240-466) |

Die anhaltende hypotensive Wirkung und eine niedrige akute Toxizität von 2-Methylmerkapto-3-zyano-4-(o-difluormethoxyphenyl)-6-phenyl-I,4-dihydropyridin zeugen von der Möglichkeit dessen Anwendung in der medizinischen Praxis als Mittel zur Behandlung der essentiellen Hypertonie.

## Beste Ausführungsform der Erfindung

Es wird empfohlen, 2-Methylmerkapto-3-zyano-4-(o-difluormethoxyphenyl)-6-phenyl-I,4-dihydropyridin nach dem Verfahren herzustellen, das die Kondensation von o-Difluor-

methoxybenzylidenazetophenon mit Zyanothioazetamid in Gegenwart von Piperidin vorsieht. Die genannten Ausgangskomponenten und Peperidin werden in äquimolaren Verhältnissen genommen. Die Reaktion wird im Medium des wasserfreien Äthylalkohols bei Zimmertemperatur und unter Normaldruck durchgeführt. Das sich bildende Piperidinsalz von I,4-Dihydropyridin wird mit Dimethylsulfat unmittelbar im Reaktionsgemisch, das heisst ohne dessen Isolierung behandelt. Das verkürzt die Dauer der Reaktionsdurchführung. Nach der Zugabe von Dimethylsulfat ins Reaktionsgemisch lässt man das Gemisch für I,5 bis 2 Stunden stehen. Der gewonnene Niederschlag wird abgetrennt und mit kaltem Äthylalkohol gewaschen. Das erhalten Endprodukt stellt einen kristallinen Stoff dar und entspricht der oben angegebenen Formel.

Zum besseren Verständnis der vorliegenden Erfindung wird ein Beispiel zur konkreten Herstellung der Verbindung angeführt.

Beispiel

Ein Gemisch aus 2,74 g (IO mMol) o-Difluormethoxybenzylidenazetophenon, I,0 g (IO mMol) Zyanothioazetamid und I,0 ml Piperidin in 6 ml wasserfreiem Äthylalkohol wird innerhalb von 2 Stunden bei Zimmertemperatur vermischt. Dem Reaktionsgemisch gibt man I,2 ml (I2,7 mMol) Dimethylsulfat zu und man filtriert ab. Nach 2 Stunden trennt man den Niederschlag ab und wäscht diesen mit kaltem Äthylalkohol und Wasser. Man erhält 3,07 g (83%) 2-Methylmerkapto--3-zyano-4-(o-difluormethoxyphenyl)-6-phenyl-I,4-dihydropyridin vom I28 bis I30°C Schm.p. (aus Äthanol).

UV-Spektrum, $\lambda_{max}$, nm (lg E): 246 (4,32), 270 (4,27), 336 (3,53).

IR-Spektrum, $\nu$ , $cm^{-I}$: I652, 2I92, 3278.

$^{I}H$-NMR-Spektrum (in DMSO-$d_6$), $\delta$ , Mg: 8,98 (I H, Singerlett, NH); 7,42-7,I8 (9 H, Multiplett, $C_6H_5$+$C_6H_4$); 7,20 (I H, Triplett, J = 74 Hz, $OCHF_2$); 4,99 (I H, Duplett, 5H); 4,67 (IH, Duplett, 4-H); 2,53 (3 H, Singulett, S-$CH_3$).

Gefunden in %: C 64,4; H 4,5; N 7,8; S 8,5.

$C_{20}H_{I6}N_2F_2OS$.

Berechnet in %: C 64,8; H 4,4; N 7,6; S 8,7.

Industrelle Anwendbarkeit

Die erfindungsgemässe Verbindung 2-Methylmerkapto-3--zyano-4-(o-difluormethoxyphenyl)-6-phenyl-I,4-dihydropyridin kann in der pharmazeutischen Industrie zur Herstellung eines Arzneimittels angewendet werden, das zur Behandlung der essentiellen Hypertonie vorgesehen ist.

PATENTANSPRUCH

2-Methylmerkapto-3-zyano-4-(o-difluormethoxyphenyl)-6-phenyl-I,4-dihydropyridin der Formel:

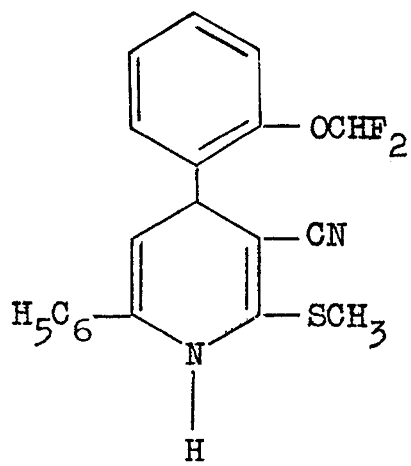

# INTERNATIONAL SEARCH REPORT 0289604

International Application No PCT/SU 86/00113

**I. CLASSIFICATION OF SUBJECT MATTER** (if several classification symbols apply, indicate all) [6]

According to International Patent Classification (IPC) or to both National Classification and IPC

$IPC^4$ C 07 D 211/90

**II. FIELDS SEARCHED**

Minimum Documentation Searched [7]

| Classification System | Classification Symbols |
|---|---|
| $IPC^4$ | C 07 D 211/90 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | GB, A, 2013186 (Institut Organicheskogo Sinteza Akademii Nauk Latviiskoi SSR et al), 8 August 1979 (08.08.79), see the claims & SU, A1, 706410, 30.12.79 DE, A1, 2900537, 12.07.79 -- | 1 |
| A | FR, A1, 2509727 (INSTITUT ORGANICHESKOGO SINTEZA AKADEMII NAUK LATVIISKOI SSR et al), 21 January 1983 (21.01.83), see the claims | 1 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

**IV. CERTIFICATION**

| Date of the Actual Completion of the International Search | Date of Mailing of this International Search Report |
|---|---|
| 12 May 1987 (12.05.87) | 6 August 1987 (06.08.87) |
| **International Searching Authority** | **Signature of Authorized Officer** |
| ISA/SU | |